# FASCICULE DE BREVET EUROPEEN

(11) **EP 2 300 473 B1**
(45) Date de publication et mention de la délivrance du brevet: **20.06.2012**
(21) Numéro de dépôt: 09769519.1
(22) Date de dépôt: 02.06.2009
(51) Int. Cl.: C07D 471/08, A61K 31/551, A61P 25/00, C07D 243/08, C07D 211/58, A61P 25/28, A61P 25/18

(54) **Sel de fumarate du 1,4-diazabicyclo[3.2.2]nonane-4-carboxylate de 4-bromophényle, ses formes cristallines, leur préparation et leur utilisation en thérapeutique**
Fumaratsalz aus 4-Bromphenyl 1,4-diazabicyclo[3.2.2]nonan-4-carboxylat, kristalline Formen davon, Zubereitung und therapeutische Verwendung
Fumarate salt of 4-bromophenyl 1,4-diazabicyclo[3.2.2]nonane-4-carboxylate, crystalline forms thereof, preparation thereof and therapeutic use thereof

(30) Priorité: 02.06.2008 FR 0802995
(43) Date de publication de la demande: 30.03.2011
(73) Titulaire: SANOFI, 75008 Paris (FR)
(72) Inventeur: ROBERT, Benoit, F-75013 Paris (FR); SALLE, Laurent, F-75013 Paris (FR)
(74) Mandataire: Morel-Pécheux, Muriel
(86) Numéro de dépôt international: PCT/FR2009/051039
(87) Numéro de publication internationale: WO 2009/156678

(56) Documents cités:
- EP-A- 1 231 212
- WO-A-2007/093600
- GOULD P L: "SALT SELECTION FOR BASIC DRUGS" INTERNATIONAL JOURNAL OF PHARMACEUTICS, ELSEVIER BV, NL, vol. 33, no. 1/03, 1 janvier 1986 (1986-01-01), pages 201-217, XP002074725 ISSN: 0378-5173

## Description

L'invention se rapporte à un nouveau sel du 1,4-diazabicyclo[3.2.2]nonane-4-carboxylate de 4-bromophényle, à ses formes cristallines, à leur préparation et à leur utilisation en thérapeutique.

Le 1,4-diazabicyclo[3.2.2]nonane-4-carboxylate de 4-bromophényle sous forme de base libre, de formule (I) ci-après, et un procédé pour sa préparation sont décrits dans le document WO 00/58311.

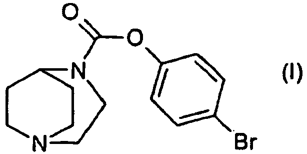

Ce composé, parmi d'autres dérivés de 1,4-diazabicyclo[3.2.2]nonane-4-carboxylate, est décrit comme un ligand des sous-unités α7 du récepteur nicotinique.

EP1231212 décrit des composés azabicycliques agonistes des récepteurs alpha -7 nicotiniques et leur utilisation en thérapeutique, Plus particulièrement dans les troubles du système nerveux central.

WO20071093600 décrit le traitement de dérivés diazabicycloalkanes agissant sur les récepteurs nicotiniques et utilisés dans le traitement des troubles du système nerveux central.

Gould P L. "Salt selection for basic drugs "International Journal of Pharmaceutics, elservier bv, nl, Vol. 33. No. 1/03. 1 janvier 1986 (1986-01-01), pages 201/217, décrit des critères de sélection de sels pour des médicaments basiques.

Le sel de chlorhydrate du 1,4-diazabicyclo[3.2.2]nonane-4-carboxylate de 4-bromophényle a par ailleurs été cité dans la littérature, par exemple dans Pichat P. et al. Neuropsychopharmacology (2007), 32(1), 17-34.

On a maintenant trouvé que la forme salifiée (2E)-but-2-ènedioate (encore appelée fumarate) de ce même composé présente des propriétés avantageuses qui la rende particulièrement adaptée à son utilisation en tant que principe actif dans un médicament.

L'invention a donc pour objet le sel de fumarate du 1,4-diazabicyclo[3.2.2]nonane-4-carboxylate de 4-bromophényle, sa préparation et son application en thérapeutique.

Le sel de fumarate du 1,4-diazabicyclo[3.2.2]nonane-4-carboxylate de 4-bromophényle est défini par la formule ci-après.

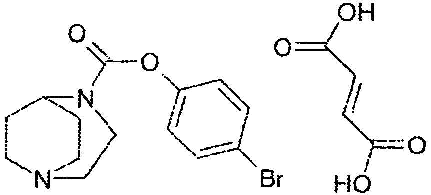

L'invention a également pour objet les deux formes polymorphiques du sel de fumarate du 1,4-diazabicyclo[3.2.2]nonane-4-carboxylate de 4-bromophényle, appelées forme I et forme II, dont les caractéristiques physico-chimiques sont décrites ci-après.

### Caractérisation de la forme I et de la forme II

### Spectre Infrarouge

Les spectres infrarouges (I.R.) des deux formes cristallines du 1,4-diazabicyclo[3.2.2]nonane-4-carboxylate de 4-bromophényle ont été enregistrés sur un spectrophotomètre à transformée de Fourier (NICOLET, Magna-IR 550) entre 4000 cm-1 et 400 cm-1, avec une résolution de 4cm-1. Les composés mélangés à du KBr (à environ 1 % de produit m/m) ont été pastillés et examinés en mode transmission.

Ces spectres sont caractérisés par les bandes d'absorption reportées dans les tableaux ci-aprés.

**Tableau spectre I.R. forme I**

| λ(cm-1) |
|---|
| 2953 |
| 2789 |
| 1735 |
| 1613 |
| 1488 |
| 624 |

**Tableau spectre I.R. forme II**

| λ(cm-1) |
|---|
| 2952 |
| 1721 |
| 1484 |
| 1200 |
| 638 |

### Diffractogramme de rayons X

Les diffractogrammes de rayons X sur poudre ont été enregistrés à partir des échantillons de poudre de la forme I et la forme II.

Les analyses ont été effectuées sur le diffractomètre D8 Advance (BRUKER-SIEMENS), équipé d'une chambre en température Anton-Paar TTK, présentant un montage en réflexion, à géométrie focalisante de type Bragg-Brentano (θ-θ).

Un tube à anticathode de cuivre fournit le rayonnement incident (λKα1 = 1,5406 Angstrom):

Enregistrement des diagrammes à température ambiante s'effectue de 2 à 40 degrés en 2θ.

**Raies caractéristiques du diffractogramme de la forme I**

| Diffraction des Rayons X de la forme I | |
|---|---|
| Pic | Angle |
| Angstrom | 2-theta° |
| d=20,46 | 4,3 |
| d=10,26 | 8,6 |
| d=6,85 | 12,9 |
| d=4,70 | 18,9 |
| d=4,47 | 19.8 |
| d=3,52 | 25,2 |

**Raies caractéristiques du diffractogramme de la forme II**

| Diffraction des Rayons X de la forme II | |
|---|---|
| Pic | Angle |
| Angstrom | 2-theta° |
| d=14,76 | 6,0 |
| d=9,54 | 9,3 |
| d=8,75 | 10,1 |
| d=4,75 | 18.7 |
| d=3,79 | 23,5 |
| d=3,69 | 24,1 |

**Paramètres de mailles de la forme I**

| Données cristallographiques et établissement de la structuré de la forme I | |
|---|---|
| Système cristallin Groupe spatial | Monoclinique P21/c |
| Dimensions de la cellule unitaire | |
| a | 22,7305Å |
| b | 7,0571Å |
| c | 12,7756 Å |
| α | 90° |
| β | 115,207° |
| γ | 90° |
| Volume | 1854,21 |
| Densité | 1,57714 |
| Nombre de molécules par maille :Z | 4 |

**Paramètres de mailles de la forme II**

| Données cristallographiques et établissement de la structure de la forme II | |
|---|---|
| Système cristallin Groupe Spatial | Monoclinique P21/c |
| Dimensions de la cellule unitaire | |
| a | 16,4735Å |
| b | 6,4183 Å |
| c | 19,4726 Å |
| α | 90° |
| β | 115,397° |
| γ | 90° |
| Volume | 1859,92 |
| Densité | 1,57230 |
| Nombre de molécules par maille : Z | 4 |

### Thermogramme

Les mesures calorimétriques différentielles en fonction de la température sont réalisées sur l'analyseur thermique Q1000 de TA instruments sous balayage d'azote.

Les échantillons à analyser ont été soumis à une programmation de température de 10°C/min de 10 à 350°C sous un balayage constant d'azote. La poudre est déposée dans des capsules en aluminium serties et percées. La quantité de produit analysé est d'environ 2 mg.

L'analyse thermodynamique différentielle effectuée sur chaque polymorphe ne présente aucun événement thermique avant la fusion.

**Tableau récapitulatif des données thermodynamiques**

| | Forme I | Forme II |
|---|---|---|
| Point de fusion (°C) +/-2°C | 176 | 175 |
| Enthalpie moyenne (J/g) +/-2J/g | 107 | 109 |

L'invention a également pour objet un procédé de préparation du sei de fumarate du 1,4-diazabicyclo[3.2.2]nonane-4-carboxylate de 4-bromophényle et de ses formes cristallines,

Conformément à l'invention, on peut préparer le composé de formule général (I) selon le procédé décrit dans la demande WO 00/58311.

Le sel de fumarate du 1,4-diazabicyclo[3.2.2]nonane-4-carboxylate de 4-bromophényle forme I peut être obtenu par réaction du 1,4-diazabicyclo[3.2.2]nonane-4-carboxylate de 4-bromophényle sous forme de base avec de l'acide fumarique dans une solvant, tel que le méthanol ou l'éthanol.

Le sel de fumarate du 1,4-diazabicyclo[3.2.2]nonane-4-carboxylate de 4-bromophényle forme Il peut être obtenu à partir du sel de fumarate du 1,4-diazabicyclo[3.2.2]nonane-4-carboxylate de 4-bromophényle forme I en présence d'un additif (sel de fumarate de 1,4-diazabicyclo[3.2.2]nonane-4-carboxylate de 2,4-dibromophényle) dans un solvant tel que le méthanol.

Le 1,4-diazabicyclo[3.2.2]nonane-4-carboxylate de 2,4-dibromophényle peut être obtenu selon le procédé décrit dans le document WO 00/58311 à partir du chloroformiate de 2,4=dibromopnényle (US2007/0270373). Le sel de fumarate de 1,4-diazabicyclo[3.2.2]nonane-4-carboxylate de [2.4]-dibromophényle peut ensuite être formé en présence d'acide fumarique dans un solvant, tel que le méthanol ou l'éthanol.

Afin d'obtenir du sel de fumarate du 1;4-diazabicyclo[3.2.2]nonane-4-carboxylate de 4-bromophényle forme Il de très bonne pureté, le sel de fumarate du 1,4-diazabicyclo[3.2.2]nonane-4-caiboxylate de 4-bromophényle forme I peut également être ensemencé avec du sel de fumarate du 1,4-diazabicyclo[3.2.2]nonane-4-carboxylate de 4-bromophényle forme II dans un solvant tel que le méthanol.

Des exemples de préparation du sel de fumarate du 1,4-diazabicyclo[3.2.2]nonane-4-carboxylate de 4-bromophényle et de ses formes cristallines sont décrits ci-après.

Dans ce qui suit, les matières de départ et les réactifs, quand leur préparation n'est pas décrite, sont disponibles dans le commerce ou décrits dans la littérature, ou bien peuvent être préparés selon des méthodes qui y sont décrites ou qui sont connues de l'Homme du métier.

### Exemple 1 : Préparation de la forme I du sel de fumarate du 1,4-diazabicyclo[3.2.2]nonane-4-carboxylate de 4-bromophényle

Dans un réacteur double enveloppe de 6 L, on place 232,45 g de 1,4-diazabicyclo[3.2.2]nonane-4-carboxylate de 4-bromophényle (WO 00/58311) dans 2331 mL d'éthanol. Le milieu réactionnel est porté à 60°C. Une solution de 87,17 g d'acide fumarique dans un 1000 mil d'éthanol et 97 mL d'eau, préalablement chauffée à 60°C. est alors ajoutée. On distille ensuite 1900 mL de solvant puis le milieu réactionnel est refroidi à 20°C en 2,5h. Après 2,5h de contact à 20°C, la forme I du sel de fumarate du 1,4-diazabicyclo[3.2.2]nonane-4-carboxylate de 4-bromophényle est filtrée, lavée par 2 fois 400 mL d'éthanol puis séchée sous vide à 50°C.
Point de fusion (DSC) : 175°C
RMN ¹H (400MHz, DMSO-d6) δ(ppm) : 1,80 (sl ^{(a)}, 2H), 2,10 (sl, 2H), 3,10 (si, 6H). 3,71 (sl, 1H), 3,84 (sl, 1H); 4,25 (sl, 0.5H), 4,40 (sl, 0.5H). 6,58 (s, 2H), 7,14 (m, 2H), 7,57 (m, 2H), 11 (sl, 2H).
(a) sl = singulet large.

### Exemple 2 : Préparation de la forme I du sel de fumarate du 1,4-diazabicyclo[3.2.2]nonane-4-carboxylate de 4-bromophényle

Dans un réacteur double enveloppe émaillé de 630 L, on place 24,26 Kg de 1,4-diazabicyclo[3.2.2]nonane-4-carboxylate de 4-bromophényle. 8,52 Kg (1,1éq) d'acide fumarique dans 197 L de méthanol. Le milieu réactionnel est agité à 65°C jusqu'à dissolution complète. Ce dernier est refroidi à 45°C en 2h puis amorcé avec 0,5% de forme I du sel de fumarate du 1,4-diazabicyclo[3.2.2]nonane-4-carboxylate de 4-bromophényle. La température est maintenue pendant une heure. Le milieu réactionnel est ensuite refroidi à 0°C en 4,5h puis maintenu 2h à cette température. La forme I du sel de fumarate du 1,4-diazabicyclo[3.2.2]nonane-4-carboxylate de 4-bromophényle est filtrée, lavée par 2 fois 24 L de méthanol froid puis séchée sous vide à 60°C.
Point de fusion (DSC) : 175°C
RMN ¹H (400MHz, DMSO-d6) δ(ppm) : 1,80 (sl^{(a)}, 2H), 2,10 (sl, 2H), 3,10 (sl, 6H), 3,71 (sl, 1H), 3,84 (sl, 1H), 4,25 (sl, 0.5H), 4,40 (sl, 0.5H), 6,58 (s, 2H), 7.14 (m, 2H). 7,57 (m, 2H), 11 (sl, 2H).
^{(a)} sl = singulet large.

### Exemple 3: Préparation de la forme II du sel de fumarate du 1,4-diazabicyclo[3.2.2]nonane-4-carboxylate de 4-bromophényle

### A) cristallisation de la forme II par ajout d'additif

On prépare une solution A de sei de fumarate du 1,4-diazabicyclo[3.2.2]nonane-4-carboxylate de 4-bromophényle forme I dans le méthanol de concentration environ égale à 30mg/ml. On chauffe aux alentours de 40°C si nécessaire afin de solubiliser l'intégralité du produit puis on filtre.

On prépare une solution B d'additif dans le méthanol de concentration environ égale à 3mg/ml. L'additif est le sel de fumarate de 1,4-diazabicyclo[3.2.2]nonane-4-carboxylate de 2,4-dibromophényle.

On mélange la solution B et la solution A afin d'obtenir une solution C de rapport (additif/1,4-diazabicyclo[3.2.2]nonane-4-carboxylate de 4-bromophényle forme I) environ égal à 0,003.

On met à évaporer à température ambiante 2ml de solution C dans un bécher sous agitation magnétique pendant environ 24h. Plusieurs béchers correspondant à plusieurs essais peuvent éventuellement être préparés.

On analyse par diffraction des rayons X sur poudre le produit D obtenu après évaporation afin de vérifier qu'il s'agit de la forme Il du sel de fumarate du 1,4-diazabicyclo[3.2.2]nonane-4-carboxylate de 4-bromophényle,

### B) Purification

On prépare une solution E de sel de fumarate du 1,4-diazabicyclo[3.2:2]nonane-4-carboxylate de 4-bromophényle forme 1 sans le méthanol de concentration environ égale à 100mg/mL On chauffe au reflux pendant environ 15 min puis on filtre. On baisse la température de la solution E à environ 10°C.

Ajouter environ 0,5% en masse de produit D (forme Il préparée à l'étape A) par rapport à la quantité de sel de fumarate du 1,4-diazabicyclo[3.2.2Jnonane-4-càrboxylate de 4-bromophényle forme I contenue dans la solution E. Or agite pendant environ 2h à environ -10°C

On filtre le produit F obtenu, puis on sèche sous balayage d'azote sec. On analyse par diffraction des rayons X sur poudre afin de vérifier qu'il s'agit de la forme II du sel de fumarate du 1,4-diazabicyclo[3.2.2]nonane-4-carbaxylate de 4-bromophényle.

Si l'on souhaite encore améliorer la pureté du sel de fumarate du 1,4-diazabicyclo[3.2.2]nonane-4-carboxylate de 4-bromophényle forme II, on peut éventuellement répéter l'étape B en ensemençant avec le produit F au lieu du produit D.

De façon inattendue, on a en effet mis en évidence que le sel de fumarate du composé de formule (i) possédait des propriétés de pureté, de stabilité et de solubilité encore améliorées par rapport au même composé sous forme de base.

En effet, il est très difficile de purifier le composé de formule (1) sous forme de base. L'étape de salification, c'est-à-dire le passage du 1,4-diazabicyclo[3.2.2]nonane-4-carboxylate de 4-bromophényle sous forme de base libre au sel de fumarate, est une étape permettant de purifier chimiquement le composé comme le montre les résultats de dosage des impuretés par CLHP (Chromatographie Liquide à Haute Performance) présentés ci-dessous (tableau 1).

### Conditions chromatographiques

Colonne : Inertsil ODS 3 150 x 4,6 mm, 5 µm, maintenue à 25°C
Phase mobile i
Tampon pH 4,5 : KH₂PO₄ 0,01 M, heptasulfonate de sodium 0,5g/l
pH ajusté à 4,5 avec KOH 1 M

| | |
|---|---|
| *Phase A :* | 90 V tampon pH 4,5 |
| | 10 V acétonitrile |
| *Phase B* : | 20 V tampon pH 4,5 |
| | 80 V acétonitrile |

*Gradient :* de 100 °fo de: phase A à 80 % de phase B en 40 min
Débit: 1.0 ml/min
Détection : UV, λ = 220 nm (trajet optique : 1 cm)

Solutions injectée solutions à 0,2 mg (base ou sel)/ml dans un mélange de 50 volumes d'eau et 50 volumes d'acétonitrile

*Remarque* : pour les dosages, les résultats obtenus sont exprimés en % (m/m) de base non dégradée (en calibrant avec ces témoins de base ou de sel non dégradés_{;} conservés à température ambiante).

**Tableau 1**

| Pureté des échantillons | | |
|---|---|---|
| | Base | Fumarate |
| Pureté par CLHP | 95,5 | 100 |

Le sel de fumarate a été obtenu avec une pureté satisfaisante contrairement à la base qui présente une pureté de 95.5%.

De plus, le sel de fumarate du 1,4-diazabicyclo[3.2.2]nonane-4-carboxylate de 4-bromophényle est plus stable que le 1,4-diazabicyclo[3.2.2]nonane-4-carboxylate de 4-bromophényle sous forme de base libre. En effet la température de fusion du 1,4-diazabicyclo[3.2.2]nonane-4-carboxylate de 4-bromophényle sous forme de base libre est de 112°C et son enthalpie de fusion de 29 kJ/mol ; tandis que la température de fusion du sel de fumarate du 1,4-diazabicyclo[3.2.2]nonane-4-carboxylate de 4-bromophényle est de 175 -176°C (respectivement forme II - forme I) et l'enthalpie de fusion est de 39 kJ/mol.

### Test de stabilité thermique à l'état solide

La base et la sel de fumarate du composé de formule (I) sont analysés après 1, 7 et 14 jours de conservation à :
- 80°C ;
- A 80°C et sous 80 % d'humidité relative (HR).

### Aspect

Les résultats sont décrits dans le tableau 2 ci-dessous.

**Tableau 2**

| Aspect - Résultats après 1 - 7 et 14 j à 80°C et 80°C/80HR | | | |
|---|---|---|---|
| **Conditions** | **Durée** | **Base** | **Fumarate** |
| Aspect Initial | - | Poudre pratiquement blanche | Poudre blanche |
| 80°C | 1 j | Jaune pâle | inchangée |
| | 7 j | Jaune pâle | inchangée |
| | 14 j | Jaune pâle | inchangée |
| 80°C/80%HR | 1 j | Jaune brune | inchangée |
| | 7 j | brune | inchangée |
| | 14 j | brune | inchangée |

Au vu des résultats obtenus ci-dessus, on constate que la base du composé de formule (I) change d'aspect sous l'effet de la chaleur et sous l'effet de la chaleur et de l'humidité, ce qui traduit une dégradation de la quaiité chimique du produit. Au contraire l'aspect du sel de fumarate du composé de formule (I) reste inchangé. Ces résultats démontrent donc une plus grande stabilité du sel de fumarate par rapport à la base du composé de formule (I) dans les conditions testées.

### Dosage du produit non dégradé et pourcentage d'impuretés

### Conditions chromatographiques

Colonne: Inertsil ODS 3 150 x 4,6 mm, 5 µm, maintenue à 25°C
Phase mobile: Tampon pH 4,5 : KH₂PO₄ 0,0.1 M, heptasulfonate de sodium 0,5g/l
pH ajusté à 4,5 avec KOH 1 M

| | |
|---|---|
| *Phase A* : | 90 V tampon pH 4,5 |
| | 10 V acétonitrile |
| *Phase B* : | 20 V tampon pH 4,5 |
| | 80 V acétonitrile |

*Gradient* : de 100 % de phase A à 80 % de phase B en 40 min
Débit : 1,0 ml/min
Détection : UV, λ = 220 nm (trajet optique: 1 cm)

Solutions injectées : solutions à 0,2 mg (base ou sel)/ml dans un mélange de 50 volumes d'eau et 50 volumes d'acétonitrile

*Remarque* : pour les dosages, les résultats obtenus sont exprimés en % (m/m) de base non dégradée (en calibrant avec des témoins de base ou de sel non dégradés, conservés à température ambiante).

Les résultats de dosage par CLHP sont résumés dans le tableau 3 ci-dessous.

**Tableau 3**

| Conditions | Durée | Base | | Fumarate | |
|---|---|---|---|---|---|
| | | Base non dégradée (%) | Somme % imp quantifiées (%surf.) | produit non dégradé (%) | Somme % imp quantifiées (% surf.) |
| initiale | - | - | 4,02 | - | 0,32 |
| 80°C | 7 j | 100 | 3,57 | 99,6 | 0,60 |
| | 14 j | 99,9 | 3,14 | 100 | 0,53 |
| 80°C/80%HR | 7 j | 98,7 | 4,37 | 99,7 | 0,74 |
| | 14 j | 98,3 | 4,53 | 100 | 0,83 |

Le sel de fumarate se dégrade donc moins que la base à la chaleur et à la chaleur en présence d'humidité.

Remarque : la somme des impuretés quantifiées peut être inférieure après stockage à 80°C sous 80 % d'humidité relative car les impuretés peuvent se dégrader en produits non détectables en UV ou non séparés par les conditions d'analyse.

Par ailleurs, au sein d'une formulation pharmaceutique, !e sel de fumarate du 1,4-diazabicyclo[3.2.2]nonane-4-carboxylate de 4-bromophényle est moins réactif vis-à-vis des excipients que le 1,4-diazabicyclo[3.2.2]nonane-4-carboxylate de 4-bromophényle sous forme de base libre. En effet, dans le cas du sel de fumarate, la liaison ionique entre l'acide fumarique et le 1,4-diazabicyclo[3.2.2]nonane-4-carboxylate de 4-bromophényle se forme au niveau de l'amine secondaire. Dans le cas de la base libre, l'absence de liaison entraine une plus grande réactivité avec les excipients lors de la formulation.

Enfin, le sel de fumarate du 1,4-diazabicyclo[3.2.2]nonane-4-carboxylate de 4-bromophényle est plus soluble que le 1,4-diazabicyclo[3.2.2]nonane-4-carboxylate de 4-bromophényle sous forme de base libre, comme le montre les analyses décrites ci-dessous.

### Tests de solubilité

Les solubilités instantanées de la base et du sel de fumarate du composé de formule (I) ont été déterminées dans les milieux suivant :
- eau ;
- tampon pH 1,2 : KH₂PO₄ 0,01 M, pH ajusté avec de l'o-acide phosphorique ;
- tampon pH 7,2 ; KH₂PO₄ 0,2 M, pH ajusté avec KOH ;
- tampon pH 9,0 : Na₂B₄O₇, 10H₂O, 0,04 M, pH ajusté avec de l'acide borique ;
- diméthylsulfoxide (DMSO).

Détermination des solubilités instantanées : des volumes successifs du milieu sont ajoutés à la prise d'essai jusqu'à la solubilisation du composé. L'agitation est faite au Vortex à température ambiante.

Les résultats sont décrits dans le tableau 4 ci-dessous. Y est également renseignée la classification correspondante selon la pharmacopée européenne.

**Tableau 4**

| Solubilités instantanées de la base et du sel de fumarate du composé de formule (I) et classification correspondante selon la pharmacopée européenne (en g de produit /l) | | | | |
|---|---|---|---|---|
| **Milieu** | **Base** | | **Fumarate** | |
| Eau | <2 | Peu soluble | ~27 | Assez soluble |
| pH 1,2 | 140 | Facilement soluble | ~16 | Assez soluble |
| pH 7,2 | <2 | Peu soluble | ~75 | Soluble |
| pH 9,0 | <2 | Peu soluble | ~68 | Soluble |
| DMSO | -75 | Soluble | ~140 | Facilement soluble |

Au vu des résultats du tableau 4, on constate que le sel de fumarate du composé de formule (I) est globalement plus soluble que le composé de formule (I) sous forme de base libre.

On a mis également en évidence que la forme fumarate du composé de formule (I) possédait des propriétés améliorées de stabilité à la température par rapport à la forme chlorhydrate de ce même composé.

En effet, sous l'effet de la température ou sous balayage d'azote, le sel de chlorhydrate du 1,4-diazabicyclo[3.2.2]nonane-4-carboxylate de 4-bromophényle a tendance à perdre sa stoechiométrie en eau. Ainsi, le contrôle et la maitrise de la phase cristalline, lors de l'étape de séchage après l'étape de salification et lors de l'étape de formulation dans le cas d'une formulation pharmaceutique du composé, présentent de nombreuses contraintes. L'isotherme de sorption-désorption d'eau montre que le chlorhydrate du composé A est présent sous une forme hémihydrate et deux formes anhydres ; cette teneur en eau difficilement maîtrisable augmente la sensibilité au séchage et rend difficile l'utilisation du chlorhydrate à l'échelle industrielle.

Au contraire, le sel de fumarate du 1,4-diazabicyclo[3.2.2]nonane-4-carboxylate de 4-bromophényle cristallise sous deux formes polymorphiques non hygroscopiques (formes I et II). La présence d'eau sous forme vapeur ou liquide n'a pas d'impact sur la forme cristalline de la forme I ou de la forme II du sel de fumarate du 1,4-diazabicyclo[3.2.2]nonane-4-carboxylate de 4-bromophényle. Cette propriété des deux formes polymorphiques du sel de fumarate du 1,4-diazabicyclo[3.2.2]nonane-4-carboxylate de 4-bromophényle est un avantage par rapport au sel de chlorhydrate du 1,4-diazabicyclo[3.2.2]nonane-4-carboxylate de 4-bromophényle.

Il ressort de ces analyses que le sel de fumarate du composé de formule (I) présente à la fois des propriétés de solubilité et de stabilité supérieures à celles du composé de formule (I) sous forme de base ou de sel de chlorhydrate, et qui le rendent particulièrement adapté à la fabrication de médicaments.

Les propriétés physico-chimiques du composé de formule (I) sous forme de sel de fumarate permettent également sa conservation dans les conditions usuelles sans précautions trop contraignantes en rapport avec la présence de lumière, la température et l'humidité.

Le sel de fumarate de 1,4-diazabicyclo[3.2.2]nonane-4-carboxylate de 4-bromophényle est un ligand des sous-unités α7 du récepteur nicotinique.

A ce titre, il peut être utilisé pour la préparation de médicaments, en particulier de médicaments destinés à traiter ou à prévenir les désordres liés à un dysfonctionnement des récepteurs nicotiniques, notamment au niveau du système nerveux central mais aussi au niveau du système périphérique.

Ces désordres comprennent les altérations cognitives, plus spécifiquement mnésiques (acquisition, consolidation et rappel), mais également les atteintes des processus attentionnels, et les troubles des fonctions exécutives liées à la maladie d'Alzheimer, au vieillissement pathologique (Age Associated Memory Impairment, AAMI) ou normal (senile dementia), au syndrome Parkinsonien, à la trisomie 21 (Down's syndrome), aux pathologies psychiatriques, en particulier les troubles cognitifs associés à la schizophrénie (Cognitive Impairment Associated with Schizophrenia, CIAS), les troubles liés au stress post-traumatique (Post-Traumatic Stress Disorder, PTSD), au syndrome alcoolique de Korsakoff, aux démences vasculaires (multi-infarct dementia, MDI), aux traumatismes crâniens.

Le composé de l'invention pourrait également être utile dans le traitement des troubles moteurs observés dans la maladie de Parkinson ou d'autres maladies neurologiques telles que la chorée de Huntington, le syndrome de Tourette, la dyskinésie tardive et l'hyperkinésie.

Il pourrait aussi présenter une activité thérapeutique neuro-protectrice vis à vis des atteintes anatomo-histo-pathologiques liées aux maladies neuro-dégénératives susnommées.

Il pourrait également être utile dans le traitement de la sclérose en planques.

Le composé de l'invention pourrait également constituer un traitement curatif ou symptomatique des accidents vasculaires cérébraux et des épisodes hypoxiques cérébraux. Il peut être utilisé dans les cas de pathologies psychiatriques : schizophrénie (symptômes positifs et/ou négatifs), troubles bipolaires, dépression, anxiété, attaques de panique, PTSD, troubles de l'attention avec hyperactivité (Attention Déficit Hyperactivity Disorder, ADHD), comportements compulsifs et obsessionnels.

Il pourrait prévenir les symptômes dus au sevrage au tabac, à l'alcool, aux différentes substances induisant une dépendance, telles que cocaïne, LSD, cannabis, benzodiazépines.

Il pourrait être utile dans le traitement de la douleur d'origine diverse (y compris les douleurs chroniques, neuropathiques ou inflammatoires).

Par ailleurs le composé de l'invention pourrait être utilisé pour le traitement de l'ischémie des membres inférieurs, de l'artérite oblitérante des membres inférieurs (PAD : peripheral arterial disease), de l'ischémie cardiaque (angor stable), de l'infarctus du myocarde, de l'insuffisance cardiaque, du déficit de cicatrisation cutanée des patients diabétiques, des ulcères variqueux de l'insuffisance veineuse, des chocs septiques.

Le composé de l'invention pourrait aussi être utilisé pour le traitement des processus inflammatoires d'origines diverses, en particulier les inflammations concernant le système nerveux central, l'inflammation pulmonaire liée aux allergies ou à l'asthme, la parodontite, la sarcoïdose, la pancréatite, les lésions de reperfusion, la polyarthrite rhumatoïde.

Le composé de l'invention pourrait également être utile dans le traitement de pathologies dermatologiques telles que le psoriasis et dans le traitement de l'asthme.

Le composé de l'invention pourrait être aussi utilisé pour le traitement de la recto-colite ulcéro-hémorragique.

Ainsi, selon un autre de ses aspects, l'invention a pour objet des médicaments qui comprennent le sel de fumarate de 1,4-diazabicyclo[3.2.2]nonane-4-carboxylate de 4-bromophényle. Ces médicaments trouvent leur emploi en thérapeutique, notamment dans le traitement et la prévention des désordres liés à un dysfonctionnement des récepteurs nicotiniques, notamment des désordres ci-dessus mentionnés.

Un autre objet de l'invention est donc l'utilisation du sel de fumarate de 1,4-diazabicyclo[3.2.2]nonane-4-carboxylate de 4-bromophényle pour la préparation d'un médicament destiné à traiter ou à prévenir les désordres liés à un dysfonctionnement des récepteurs nicotiniques, notamment les désordres ci-dessus mentionnés.

Selon un autre de ses aspects, la présente invention concerne des compositions pharmaceutiques comprenant, en tant que principe actif, le sel de fumarate de 1,4-diazabicyclo[3.2.2]nonane-4-carboxylate de 4-bromophényle. Ces compositions pharmaceutiques contiennent une dose efficace du sel de fumarate de 1,4-diazabicyclo[3.2.2]nonane-4-carboxylate de 4-bromophényle, ainsi qu'au moins un excipient pharmaceutiquement acceptable. Lesdits excipients sont choisis selon la forme pharmaceutique et le mode d'administration souhaités, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le sel de fumarate de 1,4-diazabicyclo[3.2.2]nonane-4-carboxylate de 4-bromophényle peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Sel de fumarate de 1,4-diazabicyclo[3.2.2]nonane-4-carboxylate de 4-bromophényle.

2. Sel de fumarate de 1,4-diazabicyclo[3.2.2]nonane-4-carboxylate de 4-bromophényle selon la revendication 1 **caractérisé en ce qu'**il est sous la forme polymorphique cristalline 1 sont le diffractogramme de rayons X sur poudre comporte au moins trois des raies caractéristiques suivantes :
| Pic | Angle |
|---|---|
| Angstrom | 2-theta° |
| d=20,46 | 4,3 |
| d=10,26 | 8,6 |
| d=6,85 | 12,9 |
| d=4,70 | 18,9 |
| d=4,47 | 19.8 |
| d=3,52 | 25,2 |

3. Sel de fumarate de 1,4-diazabicyclo[3.2.2]nonane-4-carboxylate de 4-bromophényle selon la revendication 2 dont le spectre infrarouge présente au moins trois des bandes d'absorption caractéristiques suivantes :
| λ(cm-1) |
|---|
| 2953 |
| 2789 |
| 1735 |
| 1613 |
| 1488 |
| 624 |

4. Sel de fumarate de 1,4-diazabicyclo[3.2.2]nonane-4-carboxylate de 4-bromophényle selon la revendication 2 dont le point de fusion est de 176°C +/-2°C et dont l'enthalpie moyenne est de 107 J/g +/-2J/g.

5. Sel de fumarate de 1,4-diazabicyclo[3.2.2]nonane-4-carboxylate de 4-bromophényle selon la revendication 1 **caractérisé en ce qu'**il est sous la forme polymorphique cristalline Il dont le diffractogramme de rayons X sur poudre comporte au moins trois des raies caractéristiques suivantes :
| Pic | Angle |
|---|---|
| Angstrom | 2-theta° |
| d=14,76 | 6,0 |
| d=9,54 | 9,3 |
| d=8,75 | 10,1 |
| d=4,75 | 18,7 |
| d=3,79 | 23,5 |
| d=3,69 | 24,1 |

6. Sel de fumarate de 1,4-diazabicyclo[3.2.2]nonane-4-carboxylate de 4-bromophényle selon la revendication 5 dont le spectre infrarouge présente au moins trois des bandes d'absorption caractéristiques suivantes :
| λ(cm-1) |
|---|
| 2952 |
| 1721 |
| 1484 |
| 1200 |
| 638 |

7. Sel de fumarate de 1,4-diazabicyclo[3.2.2]nonane-4-carboxylate de 4-bromophényle selon la revendication 5 dont le point de fusion est de 175°C +/-2°C et dont l'enthalpie moyenne est de 109 J/g +/-2J/g.

8. Procédé de préparation du sel de fumarate de 1,4-diazabicyclo[3.2.2]nonane-4-carboxylate de 4-bromophényle forme I, **caractérisé en ce que** l'on fait réagir le 1,4-diazabicyclo[3.2.2]nonane-4-carboxylate de 4-bromophényle sous forme de base libre avec de l'acide fumarique dans un solvant.

9. Procédé de préparation du sel de fumarate de 1,4-diazabicyclo[3.2.2]nonane-4-carboxylate de 4-bromophényle forme II, **caractérisé en ce que** le sel de fumarate du 1,4-diazabicyclo[3.2.2]nonane-4-carboxylate de 4-bromophényle forme I est mis en présence du sel de fumarate de 1,4-diazabicyclo[3.2.2]nonane-4-carboxylate de 2,4-dibromophényle dans un solvant.

10. Médicament, **caractérisé en ce qu'**il comprend le sel de fumarate de 1,4-diazabicyclo[3.2.2]nonane-4-carboxylate de 4-bromophényle.

11. Composition pharmaceutique, **caractérisée en ce qu'**elle comprend, à titre de principe actif, le sel de fumarate de 1,4-diazabicyclo[3.2.2]nonane-4-carboxylate de 4-bromophényle, ainsi qu'au moins un excipient pharmaceutiquement acceptable.

12. Utilisation du sel de fumarate de 1,4-diazabicyclo[3.2.2]nonane-4-carboxylate de 4-bromophényle pour la préparation d'un médicament destiné à traiter ou à prévenir les désordres liés à un dysfonctionnement des récepteurs nicotiniques.

13. Utilisation selon la revendication 12, pour la préparation d'un médicament destiné à traiter ou à prévenir les troubles des fonctions exécutives liées à la maladie d'Alzheimer.

14. Utilisation selon la revendication 12, pour la préparation d'un médicament destiné à traiter ou à prévenir les troubles cognitifs associés à la schizophrénie.

## Claims

1. Fumarate salt of 4-bromophenyl 1,4-diazabicyclo[3.2.2]nonane-4-carboxylate.

2. Fumarate salt of 4-bromophenyl 1,4-diazabicyclo[3.2.2]nonane-4-carboxylate according to Claim 1, **characterized in that** it is in the crystalline polymorphic form I, the powder X-ray diffractogram of which comprises at least three of the following characteristic lines:
| Peak | Angle |
|---|---|
| Angstrom | 2-theta° |
| d=20.46 | 4.3 |
| d=10.26 | 8.6 |
| d=6.85 | 12.9 |
| d=4.70 | 18.9 |
| d=4.47 | 19.8 |
| d=3.52 | 25.2 |

3. Fumarate salt of 4-bromophenyl 1,4-diazabicyclo[3.2.2]nonane-4-carboxylate according to Claim 2, the infrared spectrum of which exhibits at least three of the following characteristic absorption bands:
| λ (cm⁻¹) |
|---|
| 2953 |
| 2789 |
| 1735 |
| 1613 |
| 1488 |
| 624 |

4. Fumarate salt of 4-bromophenyl 1,4-diazabicyclo[3.2.2]nonane-4-carboxylate according to Claim 2, the melting point of which is 176°C +/-2°C and the mean enthalpy of which is 107 J/g +/- 2 J/g.

5. Fumarate salt of 4-bromophenyl 1,4-diazabicyclo[3.2.2]nonane-4-carboxylate according to Claim 1, **characterized in that** it is in the crystalline polymorphic form II, the powder X-ray diffractogram of which comprises at least three of the following characteristic lines:
| Peak | Angle |
|---|---|
| Angström | 2-theta° |
| d=14.76 | 6.0 |
| d=9.54 | 9.3 |
| d=8.75 | 10.1 |
| d=4.75 | 18.7 |
| d=3.79 | 23.5 |
| d=3.69 | 24.1 |

6. Fumarate salt of 4-bromophenyl 1,4-diazabicyclo[3.2.2]nonane-4-carboxylate according to Claim 5, the infrared spectrum of which exhibits at least three of the following characteristic absorption bands:
| λ (cm⁻¹) |
|---|
| 2952 |
| 1721 |
| 1484 |
| 1200 |
| 638 |

7. Fumarate salt of 4-bromophenyl 1,4-diazabicyclo[3.2.2]nonane-4-carboxylate according to Claim 5, the melting point of which is 175°C +/-2°C and the mean enthalpy of which is 109 J/g +/- 2 J/g.

8. Process for the preparation of the fumarate salt of 4-bromophenyl 1,4-diazabicyclo[3.2.2]nonane-4-carboxylate, form I, **characterized in that** 4-bromophenyl 1,4-diazabicyclo[3.2.2]nonane-4-carboxylate in the free base form is reacted with fumaric acid in a solvent.

9. Process for the preparation of the fumarate salt of 4-bromophenyl 1,4-diazabicyclo[3.2.2]nonane-4-carboxylate, form II, **characterized in that** the fumarate salt of 4-bromophenyl 1,4-diazabicyclo[3.2.2]nonane-4-carboxylate, form I, is brought together with the fumarate salt of 2,4-dibromophenyl 1,4-diazabicyclo[3.2.2]nonane-4-carboxylate in a solvent.

10. Medicament, **characterized in that** it comprises the fumarate salt of 4-bromophenyl 1,4-diazabicyclo[3.2.2]nonane-4-carboxylate.

11. Pharmaceutical composition, **characterized in that** it comprises, as active principle, the fumarate salt of 4-bromophenyl 1,4-diazabicyclo-[3.2.2]nonane-4-carboxylate, and also at least one pharmaceutically acceptable excipient.

12. Use of the fumarate salt of 4-bromophenyl 1,4-diazabicyclo-[3.2.2]nonane-4-carboxylate in the preparation of a medicament intended to treat or to prevent disorders related to a dysfunctioning of the nicotinic receptors.

13. Use according to Claim 12, in the preparation of a medicament intended to treat or to prevent disorders of the executive functions related to Alzheimer's disease.

14. Use according to Claim 12, in the preparation of a medicament intended to treat or to prevent cognitive impairment associated with schizophrenia.

## Patentansprüche

1. Fumaratsalz von 4-Bromphenyl-1,4-diazabicyclo[3.2.2]nonan-4-carboxylat.

2. Fumaratsalz von 4-Bromphenyl-1,4-diazabicyclo[3.2.2]nonan-4-carboxylat nach Anspruch 1, **dadurch gekennzeichnet, dass** es in der polymorphen Kristallform I vorliegt, deren Röntgenpulver-Diffraktogramm mindestens drei der folgenden charakteristischen Linien aufweist:
| Maximum | Winkel |
|---|---|
| Ängström | 2-theta ° |
| d = 20,46 | 4,3 |
| d = 10,26 | 8, 6 |
| d = 6,85 | 12,9 |
| d = 4,70 | 18, 9 |
| d = 4,47 | 19,8 |
| d = 3,52 | 25,2 |

3. Fumaratsalz von 4-Bromphenyl-1,4-diazabicyclo[3.2.2]nonan-4-carboxylat nach Anspruch 2, dessen Infrarotspektrum mindestens drei der folgenden charakteristischen Absorptionsbanden aufweist:
| λ (cm⁻¹) |
|---|
| 2953 |
| 2789 |
| 1735 |
| 1613 |
| 1488 |
| 624 |

4. Fumaratsalz von 4-Bromphenyl-1,4-diazabicyclo[3.2.2]nonan-4-carboxylat nach Anspruch 2, dessen Schmelzpunkt 176 °C +/- 2°C beträgt, und dessen mittlere Enthalpie 107 J/g +/- 2 J/g beträgt.

5. Fumaratsalz von 4-Bromphenyl-1,4-diazabicyclo[3.2.2]nonan-4-carboxylat nach Anspruch 1, **dadurch gekennzeichnet, dass** es in der polymorphen Kristallform II vorliegt, deren Röntgenpulver-Diffraktogramm mindestens drei der folgenden charakteristischen Linien aufweist:
| Maximum | Winkel |
|---|---|
| Ängström | 2-theta ° |
| d = 14,76 | 6, 0 |
| d = 9,54 | 9, 3 |
| d = 8, 75 | 10,1 |
| d = 4,75 | 18,7 |
| d = 3, 79 | 23, 5 |
| d = 3, 69 | 24,1 |

6. Fumaratsalz von 4-Bromphenyl-1,4-diazabicyclo[3.2.2]nonan-4-carboxylat nach Anspruch 5, dessen Infrarotspektrum mindestens drei der folgenden charakteristischen Absorptionsbanden aufweist:
| λ (cm⁻¹) |
|---|
| 2952 |
| 1721 |
| 1484 |
| 1200 |
| 638 |

7. Fumaratsalz von 4-Bromphenyl-1,4-diazabicyclo[3.2.2]nonan-4-carboxylat nach Anspruch 5, dessen Schmelzpunkt 175 °C +/- 2°C beträgt, und dessen mittlere Enthalpie 109 J/g +/- 2 J/g beträgt.

8. Verfahren zur Herstellung des Fumaratsalzes von 4-Bromphenyl-1,4-diazabicyclo[3.2.2]nonan-4-carboxylat, Form I, **dadurch gekennzeichnet, dass** man 4-Bromphenyl-1,4-diazabicyclo[3.2.2]nonan-4-carboxylat in Form der freien Base mit Fumarsäure in einem Lösungsmittel umsetzt.

9. Verfahren zur Herstellung des Fumaratsalzes von 4-Bromphenyl-1,4-diazabicyclo[3.2.2]nonan-4-carboxylat, Form II, **dadurch gekennzeichnet, dass** man das Fumaratsalz von 4-Bromphenyl-1,4-diazabicyclo[3.2.2]nonan-4-carboxylat, Form I, mit dem Fumaratsalz von 2,4-Dibromphenyl-1,4-diazabicyclo[3.2.2]nonan-4-carboxylat in einem Lösungsmittel zusammenbringt.

10. Arzneimittel, **dadurch gekennzeichnet, dass** es das Fumaratsalz von 4-Bromphenyl-1,4-diazabicyclo[3.2.2]nonan-4-carboxylat umfasst.

11. Pharmazeutische Zusammensetzung, **dadurch gekennzeichnet, dass** sie als Wirkstoff das Fumaratsalz von 4-Bromphenyl-1,4-diazabicyclo[3.2.2]nonan-4-carboxylat sowie mindestens einen pharmazeutisch annehmbaren Excipienten umfasst.

12. Verwendung des Fumaratsalzes von 4-Bromphenyl-1,4-diazabicyclo[3.2.2]nonan-4-carboxylat zur Herstellung eines Arzneimittels zur Behandlung oder Vorbeugung der Störungen in Verbindung mit einer Dysfunktion nikotinischer Rezeptoren.

13. Verwendung nach Anspruch 12 zur Herstellung eines Arzneimittels zur Behandlung oder Vorbeugung der Störungen der ausführenden Funktionen in Verbindung mit der Alzheimer-Krankheit.

14. Verwendung nach Anspruch 12 zur Herstellung eines Arzneimittels zur Behandlung oder Vorbeugung der kognitiven Störungen in Verbindung mit Schizophrenie.
